(19) Europäisches Patentamt
European Patent Office
Office européen des brevets

(11) Numéro de publication: **0 029 000**
**A1**

(12) # DEMANDE DE BREVET EUROPEEN

(21) Numéro de dépôt: **80810240.4**

(22) Date de dépôt: **31.07.80**

(51) Int. Cl.³: **C 07 C 103/46**
C 07 C 127/15, C 07 C 91/26

(30) Priorité: **03.08.79 CH 7126/79**

(43) Date de publication de la demande:
**20.05.81 Bulletin 81/20**

(84) Etats Contractants Désignés:
**AT BE DE FR GB IT NL SE**

(71) Demandeur: **INTERCO S.A.**
**30, rue St-Pierre**
**CH-1700 Fribourg(CH)**

(72) Inventeur: **Baudet, Pierre**
**15, Chemin de Passoret**
**CH-1227 Geneve(CH)**

(54) **Sels mixtes d'un métal alcalino-terreux et de diméthylamino-éthanol d'acides N-acyl-glutamiques et N-acyl-aspartiques.**

(57) Sels mixtes de magnésium ou de calcium et de diméthylamino-éthanol de l'acide N-acétyl-L- glutamique ou de l'acide N-carbamyl-L-glutamique ou de l'acide N-carbamyl-L-aspartique préparés à partir de MgO respectivement CaO, de diméthylaminoéthanol et de dérivés appropriés des acides glutamiques ou aspartiques

EP 0 029 000 A1

Sels mixtes d'un métal alcalino-terreux et de diméthylamino-éthanol d'acides N-acyl-glutamiques et N-acyl-aspartiques.

DESCRIPTION

Les sels mixtes de magnésium ou de calcium et de diméthylamino-éthanol des acides α aminés dicarboxyliques et N-acylés de la formule :

$$A \quad (R-NH-\underset{\underset{\overset{\|}{O}}{CO-}}{CH}-(CH_2)_n-\underset{\overset{\|}{O}}{C}O-)_2\ M^{++}((CH_3)_2\overset{+}{N}H-CH_2-CH_2OH)_2$$

dans laquelle $R = CH_3\underset{\overset{\|}{O}}{C}-$, $n = 2$, $M^{++} =$ Mg ou Ca

dans laquelle $R = NH_2\underset{\overset{\|}{O}}{C}-$, $n = 2$, $M^{++} =$ Mg ou Ca

dans laquelle $R = NH_2\underset{\overset{\|}{O}}{C}-$, $n = 1$, $M^{++} =$ Mg ou Ca

se présentent sous forme cristallisée, comme hydrate et peu hydroscopique.

Les sels mixtes de l'invention ont des applications intéressantes en thérapeutique lorsque l'organisme humain est en pleine croissance ou lorsqu'il est en déficience du

fait de l'âge. En effet, les trois constituants des sels mixtes de l'invention jouent un rôle métabolite réparateur: (1) l'acide N-acétyl-L-glutamique est le précurseur de l'arginine via l'acide N-acétyl-L-glutamique semialdéhyde, la N-acétyl-ornithine, l'ornithine et l'acide arginosuccinique; il est l'activateur de l'enzyme carbamyl-phosphate-synthétase qui fournit l'acide carbamyl-phosphorique, point de départ chez l'homme de la biosynthèse des pyrimidines constituants des acides ribo et désoxy-ribo-polynucléiques, ceci par formation intermédiaire de l'acide N-carbamyl-L-aspartique.

(2) l'acide N-carbamyl-L-aspartique fournit le point de départ de la biosynthèse des pyrimidines aboutissant aux nucléotides et aux acides ribo et désoxyribo-nucléiques. L'apport de l'acide carbamyl-L-aspartique est très important lorsque l'organisme humain est appauvri en carbamyl-aspartate transférase, enzyme nécessaire à sa biosynthèse à la suite de déficiences physiologiques de l'organisme en pleine croissance ou en sénescence. Le diméthylamino-éthanol à la suite de la méthylation catalysée par la méthionine-méthyl-transférase fournit de la choline, dont l'action sur le métabolisme empêche l'infiltration graisseuse du foie, dont l'acétylation fournit le médiateur de l'influx nerveux, l'acétylcholine et qui entre dans la composition de phospholipides des membranes cellulaires, comme les lécithines, les céphalines et les plasmagènes. Enfin le cation magnésium est nécessaire à l'action de la carbamyl-aspartate-transférase et à bien d'autres activités enzymatiques.

Il s'en suit que les sels mixtes de l'invention jouent par le moyen de tous leurs constituants une activité réparatrice et suppléante de métabolismes fondamentaux, dont la carence

est pathologique. Les sels mixtes de l'invention seront donc utilisés avantageusement en thérapeutique dans l'asthénie, la diminution de l'activité intellectuelle, les déficits moteurs divers, les scléroses vasculaires et leurs séquelles, les apathies provenant de l'usage de sédatifs, la confusion mentale, troubles de la mémoire etc...

Leurs mises en oeuvre pharmaceutiques pourra se faire par ex. avec de l'amidon, du talc etc...

Exemple 1

Sel mixte de magnésium et de diméthylamino-éthanol de l'acide N-acétyl-L-glutamique

A une suspension de 18,9 g d'acide N-acétyl-L-glutamique dans de l'eau on ajoute 4,0 g de MgO; après une heure d'agitation, la mise en solution est complète, on ajoute alors encore 18,9 g d'acide N-acétyl-L-glutamique,lorsque la dissolution est totale le pH est de 3-4. On concentre la solution jusqu'à l'obtention d'une pâte cristalline et cristallise le sel acide de magnésium dans de l'éthanol.

$(N\text{-acétyl-L-glutamate})_2.Mg.2H_2O$

% Mg trouvé = 4,87 %

% eau de cristallisation trouvé = 7,26%

A une suspension de 40 g du sel acide de magnésium précédent, on ajoute 17,8 g de diméthylamino-éthanol et sous agitationla mise en solution s'opère graduellement avec une exothermine manifeste. Lorsque la solution est homogène, on évapore le solvant et cristallise le sel mixte (pH neutre) dans de l'acétone.

$(N\text{-acétyl-L-glutamate})_2Mg(\text{diméthylamino-éthanol})_2.2H_2O$

$C_{22}H_{42}N_4O_{12}Mg.2H_2O(614)$ Mg : 3,88% , $H_2O$ crist.: 5,92 %

C : 42,85 % N : 9,05 %

Exemple 2

Sel mixte de calcium et de diméthylamino-éthanol de l'acide N-acétyl-L-glutamique.

A une suspension de 37,8 g d'acide N-acétyl-L-glutamique dans de l'eau, on ajoute 5,6 g de CaO, lorsque la dissolution est achevée, on évapore le solvant sous pression réduite et cristallise le sel acide de calcium dans de l'acétone par grattage et lente agitation; pH 3-4.

(N-acétyl-L-glutamate)$_2$ Ca. $3H_2O$

% de Ca : 19,85 , $H_2O$ de cristallisation : 14,79 %

A une solution de 13,5 g du sel acide de calcium dans du méthanol on ajoute 8,9 g de diméthylamino-éthanol, on évapore et cristallise le sel mixte dans un mélange d'acétone et d'éthanol.

(N-acétyl-L-glutamate)$_2$C$_a$ (diméthylamino-éthanol)$_2$.$2H_2O$

% de C$_a$ : 6,41 % , % eau de cristallisation : 5,65 %

Exemple 3

Sel mixte de magnésium et de diméthylamino-éthanol de l'acide N-carbamyl-L-aspartique.

A une suspension de 17,6 g d'acide N-carbamyl-L-aspartique dans de l'eau on ajoute 2,o g de MgO. On porte la température du milieu de réaction à 58$^o$, afin de prévenir la cristallisation du sel; lorsque la solution est homogène et de pH 3-4, on évapore et cristallise le sel acide de magnésium dans de l'éthanol.

(N-carbamyl-L-aspartate)$_2$Mg. $2H_2O$

% Mg : 5,72 % , teneur en $H_2O$ de cristallisation : 8,12 %

A une suspension de 20,5 g du sel acide de magnésium précédent dans du méthanol, on ajoute 8,9 g de diméthylamino-éthanol, et agite durant 15 heures; on filtre le sel mixte à l'état cristallisé.

(N-carbamyl-L-aspartate)$_2$Mg (diméthylamino-éthanol)$_2$.2 $H_2O$
% Mg : 3,97 % , teneur en $H_2O$ de cristallisation : 6,19 %

Exemple 4

Sel mixte de calcium et de diméthylamino-éthanol de l'acide N-carbamyl-L-aspartique.

A une suspension de 34,2 g d'acide N-carbamyl-L-aspartique dans de l'eau on ajoute 5,6 g de CaO; lorsque la dissolution est complète on évapore et cristallise dans de l'acétone.

(N-carbymal-L-aspartate)$_2$$C_a$ . 3$H_2O$
% de Ca : 9,12 % , % eau de cristallisation : 12,7 %

On porte 4,36 g du sel acide de calcium précédent dans du méthanol contenant 1,78 g de diméthylamino-éthanol; lorsque le pH est à environ 7-7,5,on évapore et cristallise le sel mixte dans de l'acétone.

(N-carbamyl-L-aspartate)$_2$ $C_a$ (diméthylamino-éthanol)$_2$2$H_2O$

% de $C_a$ : 4,1 % , % $H_2O$ de cristallisation : 6,22 %

## REVENDICATIONS

1. Sels mixtes d'un métal alcalino-terreux et de diméthylamino-éthanol d'acides N-acyl-L-glutamiques et d'acide N-ayl-L-aspartique de la formule générale :

I

$$(R-NH-\underset{\underset{O}{CO^-}}{CH}-(CH_2)_n-\underset{\overset{\|}{O}}{C}O^-)_2 \quad M^{++}((CH_3)_2NH^+-CH_2-CH_2OH)_2$$

dans laquelle R est le reste $CH_3-\underset{\overset{\|}{O}}{C}-$, n =2, $M^{++}$= Mg ou Ca

dans laquelle R est le reste $NH_2-\underset{\overset{\|}{O}}{C}-$, n=2, $M^{++}$= Mg ou Ca

dans laquelle R est le reste $NH_2-\underset{\overset{\|}{O}}{C}-$, n=1, $M^{++}$= Mg ou Ca

2. Procédé selon la revendication 1, de préparation du sel mixte de magnésium et de diméthylamino-éthanol de l'acide N-acétyl-L-glutamique, caractérisé par ce que l'on fait réagir deux équivalents d'acide N-acétyl-L-glutamique avec un équivalent de MgO dans de l'eau par ex., que l'on concentre la solution obtenue et cristallise le sel acide de magnésium dans de l'éthanol par ex.,

caractérisé par ce que l'on fait réagir ce sel acide avec

deux équivalents de diméthylamino-éthanol dans un solvant convenable comme par ex. de l'éthanol ou de l'acétonitrile ou du méthanol. On filtre le produit cristallisé selon la nature du solvant ou on élimine le solvant par évaporation et cristallise le sel mixte dans de l'acétone.

3.Procédé selon la revendication 1, de préparation du sel mixte de calcium et de diméthylamino-éthanol de l'acide N-acétyl-L-glutamique, caractérisé par ce que l'on fait réagir deux équivalents d'acide N-acétyl-L-glutamique dans de l'eau par ex., que l'on isole le sel acide de calcium de l'acide N-acétyl-L-glutamique, après l'adjonction d'un équivalent de CaO et cristallise le sel acide dans de l'acétone,

caractérisé par ce que l'on fait réagir ce sel acide de calcium avec deux équivalents de diméthylamino-éthanol dans un solvant convenable, comme du méthanol et cristalli se le sel mixte comme hydrate dans de l'acétone par ex.

.4.Procédé selon la revendication 1, de préparation du sel mixte de magnésium et de diméthylamino-éthanol de l'acide N-carbamyl-L-glutamique, caractérisé par ce que l'on fait réagir deux équivalents d'acide N-carbamyl-L-glutamique avec un équivalent de MgOdans un solvant comme de l'eau, concentre et cristallise le sel acide de magnésium dans de l'éthanol par ex., caractérisé par ce que l'on fait réa gir ce sel acide de magnésium avec deux équivalents de diméthylamino-éthanol dans un solvant convenable comme par ex. du méthanol et que l'on cristallise le sel mixte dans de l'éthanol par ex.

5.Procédé selon la revendication 1, de préparation du sel mixte de calcium et de diméthylamino-éthanol de l'acide

N-carbamyl-L-glutamique, caractérisé par ce que l'on fait réagir deux équivalents d'acide N-carbamyl-L-glutamique avec un équivalent de CaO dans un solvant comme de l'eau, concentre et cristallise le sel acide de calcium dans de l'acétone par ex.,

caractérisé par ce que l'on fait réagir le sel acide de calcium avec deux équivalents de diméthylamino-éthanol dans un solvant convenable comme du méthanol par ex. et cristallise le sel mixte dans de l'acétone.

6.Procédé selon la revendication 1, de préparation du sel mixte de magnésium et de diméthylamino-éthanol de l'acide N-carbamyl-L-aspartique, caractérisé par ce que l'on fait réagir deux équivalents d'acide N-carbymyl-L-aspartique avec un équivalent de MgO dans un solvant convenable comme l'eau, concentre et cristallise le sel acide de magnésium dans de l'éthanol ou de l'acétone,

caractérisé par ce que l'on fait réagir le sel acide de magnésium avec deux équivalents de diméthylamino-éthanol dans du méthanol par ex. et cristallise le sel mixte dans de l'éthanol.

7.Procédé selon la revendication 1, de préparation du sel mixte de calcium et de diméthylamino-éthanol de l'acide N-carbamyl-L-aspartique, caractérisé par ce que l'on fait réagir deux équivalents d'acide N-carbamyl-L-aspartique avec un équivalent de CaO, dans de l'eau et cristallise le sel acide de calcium dans de l'acétone,

caractérisé par ce que l'on fait réagir le sel acide de calcium avec deux équivalents de diméthylamino-éthanol dans du méthanol et cristallise le sel mixte dans de l'acétone.

8. Procédé selon la revendication 1, de préparation des sels mixtes de magnésium ou de calcium et de diméthylamino-éthanol des acides N-acétyl-L-glutamique, N-carbamyl-L-glutamique et N-carbamyl-L-aspartique de la formule I sans au préalable isoler les sels acides de magnésium ou de calcium, caractérisé par ce que l'on fait réagir deux équivalents des acides N-acylés avec un équivalent de MgO ou de CaO dans du méthanol par ex. puis ajoute à cette solution ou suspension à caractère acide deux équivalents de diméthylamino-éthanol. Les sels mixtes sont cristallisés comme indiqué dans les revendications 2 à 7.

0029000

Office européen des brevets

# RAPPORT DE RECHERCHE EUROPEENNE

Numéro de la demande

EP 80 81 0240.4

## DOCUMENTS CONSIDERES COMME PERTINENTS

| Catégorie | Citation du document avec indication, en cas de besoin, des parties pertinentes | Revendication concernée |
|---|---|---|
| A | FR - M - 2487 (INTERCO FRIBOURG)<br>-- | |
| A | FR - M - 7642 (SOCIETA PRODOTTI ANTIBIOTICI S.P.A.)<br>-- | |
| A | US - A - 2 941 924 (STE DES USINES CHIMIQUES RHONE-POULENC)<br>-- | |
| A | CH - A - 551 377 (CENTRES D'ETUDES POUR L'INDUSTRIE PHARMACEUTIQUE)<br>---- | |

**CLASSEMENT DE LA DEMANDE (Int. Cl.3)**

C 07 C 103/46
C 07 C 127/15
C 07 C 91/26

**DOMAINES TECHNIQUES RECHERCHES (Int. Cl.3)**

C 07 C 91/26
C 07 C 103/46
C 07 C 127/15

**CATEGORIE DES DOCUMENTS CITES**

- X: particulièrement pertinent
- A: arrière-plan technologique
- O: divulgation non-écrite
- P: document intercalaire
- T: théorie ou principe à la base de l'invention
- E: demande faisant interférence
- D: document cité dans la demande
- L: document cité pour d'autres raisons
- &: membre de la même famille, document correspondant

X Le présent rapport de recherche a été établi pour toutes les revendications

| Lieu de la recherche | Date d'achèvement de la recherche | Examinateur |
|---|---|---|
| Berlin | 03-12-1980 | STOOS |